# EUROPÄISCHE PATENTANMELDUNG

(11) **EP 0 856 731 A2**
(43) Veröffentlichungstag der Anmeldung: **05.08.1998**
(21) Anmeldenummer: 98100299.1
(22) Anmeldetag: 09.01.1998
(51) Int. Cl.: G01N 15/14

(54) **Verfahren und Vorrichtung zur Bestimmung der Grössenverteilung von verschiedenartigen Partikeln in einer Probe**

(30) Priorität: 10.01.1997 DE 19700648
(71) Anmelder: BASF AKTIENGESELLSCHAFT, 67056 Ludwigshafen (DE)
(72) Erfinder: Esser, Anton, Dr., 68199 Mannheim (DE); Runge, Frank, Dr., 67133 Maxdorf (DE); Kaub, Hans-Peter, 67122 Altrip (DE)
(74) Vertreter: Isenbruck, Günter, Dr.

(57) **Zusammenfassung**

Verfahren und Vorrichtung zur Bestimmung der Größenverteilung von mindestens zwei verschiedenen Arten von fluoreszierenden oder fluoreszent angefärbten Teilchen in einer Probe (8), wobei Licht (7) in die Probe (8) eingestrahlt wird und Streulicht (16) und Fluoreszenzlicht (11) aus der Probe aufgenommen und ausgewertet (23, 24, 25) wird.

## Beschreibung

Die vorliegende Erfindung bezieht sich auf ein Verfahren und auf eine Vorrichtung zur Bestimmung der gemeinsamen Größenverteilung von Partikeln verschiedener Art in einer Probe.

Im Bereich der Papierherstellung fallen zum Beispiel wäßrige Suspensionen an, die Störstoffteilchen verschiedener Art und Größe enthalten. Diese Störstoffteilchen sind häufig hydrophob und klebrig. Sie können zu Ablagerungen in Maschinen führen, die in teueren Standzeiten beseitigt werden müssen. Solche Ablagerungen bestehen zum Beispiel aus Polyvinylacetat, Polystyrol, Polyacrylat, Wachs, Paraffin, Naturkautschuk oder anderen Substanzen, die häufig von recycelten Klebebändern, Schmelzklebebrücken, Selbstklebekuverts oder von Streichereiausschuß oder bitumiertem oder paraffiniertem Papier stammen. Um solchen Ablagerungen vorzubeugen, versucht man, die Störstoffe entweder herauszufiltern oder auszufällen, oder man mengt dem Papierstoff chemische Zusätze bei, die die Störstoffteilchen fein verteilt an die Holzfasern im Papierstoff binden und so die Ablagerung in den Maschinen verhindern. Die Qualität und die Wirtschaftlichkeit der Papierherstellung hängt dabei von Auswahl und Dosierung des Zusatzes ab. Bei Schwankungen in der Art, Menge und Größe der Störstoffteilchen muß auch die Art und Menge des zugegebenen Zusatzes verändert werden. Das setzt wiederum voraus, daß man Art, Menge und Größe der Störstoffteilchen möglichst genau kennt. Besondere Bedeutung hat dabei die Kenntnis der Größenverteilung der Störstoffpartikel.

Mit herkömmlichen Untersuchungsverfahren, etwa der Röntgen-Mikroanalyse, der Infrarot-Spektralphotometrie oder der Gel-Permeationschromatographie, wie sie bei R. Wilken und J. Strauss, "Grundlegende Untersuchungen über klebende Verunreinigungen im wiederverwendeten Altpapier", Mitteilungen aus dem Papiertechnischen Institut der Papiertechnischen Stiftung, Band 11-12 (1984), Seite 292 ff., im Überblick beschrieben sind, kann die Art der Störstoffteilchen, also ihre chemische Zusammensetzung im Labor bestimmt werden. Es lassen sich auch qualitative Aussagen über Konzentration und Teilchengrößenverteilung treffen. Diese Verfahren haben aber allesamt den Nachteil, daß sie relativ zeit- und arbeitsaufwendig sind und damit für die unmittelbare Überwachung von Störstoffveränderungen und der Wirkung von Zusätzen auf die Bindung der Störstoffe an den Papierstoff während des Produktionszyklus nicht geeignet sind.

Ein anderes Verfahren zur Bestimmung der Teilchengrößenverteilung von Störstoffteilchen wird bei T. Kröhl, P. Lorencak, A. Gierulski, H. Eipel und D. Horn, "A new laser-optical method for counting colloidally dispersed pitch", Nordic Pulp and Paper Research Journal, Band 9 (1994), Nr.1, Seite 26 ff. beschrieben. Bei diesem Verfahren werden Störstoffteilchen in einer Küvette durch hydrodynamische Fokussierung vereinzelt und mit einem Fluoreszenzfarbstoff angefärbt. Anschließend wird Laserlicht in die Probe mit den vereinzelten Störstoffteilchen eingestrahlt und von diesen ausgesandtes Fluoreszenzlicht aufgenommen. Aus der Intensität der Fluoreszenzsignale kann man dann auf die Teilchengrößenverteilung schließen. Dieses Verfahren liefert allerdings nur dann eine hinreichend genaue Teilchengrößenverteilung, wenn die Probe entweder nur eine Teilchenart enthält, oder zwar mehrere Teilchenarten aufweist, diese aber über eine annähernd gleiche Anfärbbarkeit für den verwendeten Fluoreszenzfarbstoff und über eine vergleichbare Quantenausbeute verfügen. Da diese Voraussetzungen in der Praxis selten gegeben sind, bietet das beschriebene fluoreszenzoptische Meßverfahren kein praktisch zuverlässiges Verfahren zur Ermittlung der Teilchengrößenverteilung in einer Probe mit mehreren verschiedenartigen Partikeln. Nachteilig ist weiterhin, daß mehrere verschiedene Partikelsorten nicht unterschieden werden können. Damit kann man auch den Zusatz in Art und Menge nicht an die jeweiligen Verhältnisse anpassen.

Ähnliche Probleme wie bei der Papierherstellung bestehen auch in vielen anderen technischen Bereichen, insbesondere bei Recycling-Verfahren, wo Störstoffe oder auch Wertstoffe möglichst genau identifiziert werden müssen, um ihre Behandlung zutreffend bestimmen zu können.

Aufgabe der vorliegenden Erfindung ist deshalb die Bereitstellung eines Verfahrens und einer Vorrichtung zur Ermittlung der Größenverteilung von mindestens zwei verschiedenartigen Partikelsorten in einer Probe. Diese Aufgabe wird durch das in den Verfahrensansprüchen beschriebene Verfahren und die in den Vorrichtungsansprüchen beschriebene Vorrichtung gelöst.

Erfindungsgemäß wird ein Verfahren zur Bestimmung der Größenverteilung von mindestens zwei Arten (A_{K}) von fluoreszierenden Teilchen (Tᵢ) in einer Probe bereitgestellt. Fluoreszierende Teilchen sind dabei solche Teilchen, die natürlicherweise oder nach einer Anfärbung mit einem Fluoreszenzfarbstoff fluoreszieren. Das erfindungsgemäße Verfahren weist mindestens folgende Schritte auf:
(a) Zunächst werden die Teilchen (Tᵢ) in der Probe vereinzelt. Das erfolgt vorzugsweise durch hydrodynamische Fokussierung der Teilchen. Dabei wird eine Suspension der zu untersuchenden Partikel kontinuierlich zusammen mit einem Wasserstrom, dem Hüllstrom, in eine Hüllstromküvette eingeleitet. Der im Vergleich zur Suspension wesentlich schneller fließende Hüllstrom verteilt die Teilchen über eine relativ weite Strecke, so daß schließlich die Teilchen überwiegend als einzelne Teilchen im Hüllstrom vorliegen.
   Nach der Vereinzelung der Teilchen wird Licht in die Probe entlang einer vorgegebenen Einstrahlungsrichtung eingestrahlt. Als Lichtquelle verwendet man vorzugsweise einen Laser.
(b) Dann wird mindestens ein Streulichtintensitätswert (S(Tᵢ)) und mindestens ein Fluoreszenzlichtintensitätswert (F(Tᵢ)) von jedem an der Lichtquelle vorbeigeführten vereinzelten Teilchen (Tᵢ) gemessen, so daß man mindestens ein Wertepaar (S(Tᵢ),F(Tᵢ)) pro Teilchen (Tᵢ) erhält. Je nachdem in welchem Umfange man zufällige Meßfehler eliminieren will, kann man auch mehrere Wertepaare pro Teilchen ermitteln. Man kann Teilchen natürlich auch nur stichprobenweise betrachten, wenn man das als hinreichend aussagekräftig betrachtet. Zur Messung des Streulichts und des Fluoreszenzlichts befinden sich entsprechende Detektoren an der Peripherie der Probe.
   Vorzugsweise wird bei dem erfindungsgemäßen Verfahren das Vorwärtsstreulicht der Probe aufgenommen, also das Streulicht, das in einem Kegel um die Einstrahlungsrichtung des Lichts aus der Probe emittiert wird. Dabei ist es vorteilhaft, das intensive Anregungslicht in der Einstrahlungsrichtung auszublenden. Vorzugsweise nimmt man die Streulichtintensitätswerte (S(Tᵢ)) deshalb in einem Hohlkegel auf, dessen innere Mantelfläche mit der Einstrahlungsrichtung einen Winkel von mindestens 5°, und dessen äußere Mantelfläche mit der Einstrahlungsrichtung einen Winkel von höchstens 50° einschließt.
   Zur Verfeinerung des Meßverfahrens kann der Streulichtmeßkegel auch in mehrere Kegelschichten, also Winkelsegmente unterteilt werden, die dann getrennt ausgewertet werden. Daneben kann auch ein Rückwärtsstreusignal oder ein 90°-Streusignal aufgezeichnet und ausgewertet werden.
(c) Im nächsten Schritt wird jedes Teilchen (Tᵢ) aufgrund der Lage seines zugehörigen Wertepaares (S(Tᵢ),F(Tᵢ)) in einem Bereich (B_{K}) in einem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, einer Teilchenart (A_{K}) zugeordnet. Dabei wird jeder Bereich (B_{K}) so bestimmt, daß er wenigstens ein lokales Maximum der Häufigkeit der Wertepaare (S(Tᵢ),F(Tᵢ)) für eine Teilchenart (A_{K}) enthält. Diese Unterscheidung der verschiedenen Teilchenarten (A_{K}) ist beim erfindungsgemäßen Verfahren erstmals möglich, weil hier die Tatsache ausgenutzt wird, daß die Intensität des Streulichts und des Fluoreszenzlichts in unterschiedlicher Weise von der Teilchenart abhängt. Ein graphischer Auftrag der Werte der Streulichtintensität gegen die zugehörigen Werte der Fluoreszenzlichtintensität führt deshalb im Regelfall zu klar unterscheidbaren Gebieten von Meßwerten, also einem lokalen Häufigkeitsmaximum der Wertepaare für die Streuung und die Fluoreszenz, wobei ein Gebiet einer bestimmten Teilchenart entspricht. Aus der Lage des Meßpunktes (S(Tⱼ), F(Tⱼ)) für ein bestimmtes Teilchen (Tⱼ) in einem bestimmten Gebiet von Meßpunkten läßt sich also seine Teilchenart bestimmen.
(d) Dann wird die relative Häufigkeit der Fluoreszenzlichtintensitätswerte (F(Tᵢ)) ermittelt und daraus die relative Teilchengrößenverteilung für jede Teilchenart (A_{K}) berechnet. Falls gewünscht, kann man hier auch bereits eine Eichung auf absolute Teilchengrößen vornehmen, was allerdings die Kenntnis eines teilchenartindividuellen Eichfaktors voraussetzt. Dies ist nach der Identifizierung der Teilchen relativ leicht möglich, weil man mit der Trennung der Teilchenarten auch die störenden Einflüsse der unterschiedlichen Anfärbbarkeit und ungleichen Quantenausbeute der verschiedenen Teilchenarten beseitigt hat. Wegen dieser Unterschiede kann aber bei herkömmlichen Verfahren aus den relativen Teilchengrößenverteilungen für die verschiedenen Teilchenarten nicht auf eine gemeinsame Größenverteilung für alle in der Probe enthaltenen Teilchenarten geschlossen werden.
(e) Anschließend werden die relativen Teilchengrößenverteilungen für die einzelnen Teilchenarten (A_{K}) mit Hilfe der Lage der Bereiche (B_{K}) in dem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, aufeinander normiert. Daraus wird dann eine gemeinsame relative Teilchengrößenverteilung für alle Teilchenarten (A_{K}) gebildet. Die Normierung kann prinzipiell mit jedem beliebigen Verfahren ausgeführt werden, solange die physikalischen Gegebenheiten angemessen erfaßt werden.

Vorzugsweise führt man, um zu dieser gemeinsamen Teilchengrößenverteilung zu gelangen, jedoch folgende weitere Schritte aus:
(a) Man wählt einen Streulichtbereich (SLB(A_{K})) von Streulichtintensitätswerten (S(Tᵢ)) für jede Teilchenart (A_{K}), der von vorbestimmter Größe ist, in dem die Häufigkeit der Wertepaare (S(Tᵢ),F(Tᵢ)) der Teilchenart (A_{K}) mindestens ein lokales Maximum aufweist, also auch die Dichte der Meßwerte zumindest lokal maximal ist. Die Bestimmung des Bereichs größter Meßwertdichte bzw. Meßwerthäufigkeit kann dabei mit Hilfe einer geeigneten Rechenregel, zweckmäßigerweise in einem Computer, geschehen, dem die Wertepaare zugänglich sind, man kann aber auch eine optische Abschätzung per Auge auf einem Bildschirm vornehmen, auf dem die Streulichtintensitätswerte (S(Tᵢ)) gegen die Fluoreszenzlichtintensitätswerte (F(Tᵢ)) aufgetragen sind. Die Größe des Bereichs wird vorher bereits bestimmt, zum Beispiel auf einige Prozent des Streulichtintensitätswertes (S(Tᵢ)), der in etwa das Zentrum des Bereichs maximaler Meßwertdichte darstellt. Letzlich muß der gewählte Bereich einfach groß genug sein, um eine sichere Mittelwertbildung durch Erfassung von genügend Wertepaaren zu erlauben und klein genug, um den Einfluß zufälliger Meßfehler möglichst gering zu halten.
(b) Dann wird ein Streulichtbereich (SLB) von Streulichtintensitätswerten (S(Tᵢ)) von vorbestimmter Größe mit einer oberen und einer unteren Bereichsgrenze bestimmt, dessen Mittelwert aus der oberen und der unteren Bereichsgrenze gleich dem Mittelwert der Mittelwerte aus den Streulichtintensitätswerten (S(Tᵢ)) in den jeweiligen Streulichtbereichen (SLB(A_{K})) ist. Der jetzt bestimmte Streulichtbereich (SLB) umfaßt für die verschiedenen Meßwertgebiete, d.h. Teilchenarten, die Bereiche größter oder zumindest sehr großer Meßwertdichte. Dieser für alle Teilchenarten einheitliche Streulichtbereich muß bestimmt werden, um die Fluoreszenzsignale der verschiedenen Teilchenarten aufeinander zu normieren, um also ein Maß für die unterschiedliche Anfärbbarkeit und Quantenausbeute der Teilchenarten zu haben. In vielen Fällen werden die Streulichtbereiche größter Meßwertdichte (SLB(A_{K})) für die einzelnen Teilchenarten (A_{K}) einander bereits weitgehend überdecken und so den gemeinsamen Streulichtbereich (SLB) bilden. Dessen Größe, also der von ihm überspannte Bereich von Streulichtintensitätswerten (S(Tᵢ)), ist vorgegeben. Die obigen Ausführungen zu den Streulichtbereichen (SLB(A_{K})) gelten diesbezüglich entsprechend.
   Statt der relativ exakten Schritte (a) und (b) kann man den Streulichtbereich (SLB) auch einfach direkt wählen, ohne vorher Streulichtbereiche (SLB(A_{K})) für die einzelnen Teilchenarten (A_{K}) zu wählen und deren Streulichtmittelwerte zu berechnen. In diesem Fall schätzt man ab, welchen Meßwertbereich der Streulichtbereich (SLB) etwa abdecken muß, um die Punkte größter Meßwertdichte für alle verschiedenen Teilchenarten (A_{K}) zu enthalten. Dabei kann man, je nach den eigenen Ansprüchen an die Genauigkeit der späteren Normierung, mehr oder weniger exakt vorgehen und sogar einen Streulichtbereich (SLB) wählen, der für eine oder mehrere Teilchenarten den Punkt größter Meßwertdichte gerade nicht enthält.
(c) Im nächsten Schritt wird jeweils ein Fluoreszenzlichtbereich (FLB(A_{K})) von Fluoreszenzlichtintensitätswerten (F(Tᵢ)) für jede Teilchenart (A_{K}) von vorbestimmter Größe bestimmt, dessen Wertepaare (S(Tᵢ),F(Tᵢ)) auch in den Streulichtbereich (SLB) fallen. Man ermittelt hier die zu den Streulichtintensitätswerten (S(Tᵢ)) im Streulichtbereich (SLB) gehörigen Fluoreszenzlichtintensitätswerte (F(Tᵢ)) für jede Teilchenart (A_{K}), d.h. für jedes Meßwertgebiet.
(d) Danach bestimmt man den Mittelwert (M(FLB(A_{K}))) der Fluoreszenzlichtintensitätswerte (F(Tᵢ)) in dem jeweiligen Fluoreszenzlichtbereich (FLB(A_{K})) für jede Teilchenart (A_{K}).
(e) Daraus errechnet man für jede Teilchenart (A_{K}) einen Normierungsfaktor (N(A_{K})), bezogen auf eine beliebige Teilchenart (A₁), wobei gilt: (N(A_{K})) = (M(FLB(A_{K})))/(M(FLB(A₁))).
(f) Als letzten Schritt setzt man die relativen Teilchengrößenverteilungen der Teilchenarten (A_{K}) mit Hilfe der Normierungsfaktoren (N(A_{K})) zueinander in Beziehung. Damit hat man aus den untereinander unvergleichbaren relativen Größenverteilungen der verschiedenen Teilchenarten (A_{K}) eine gemeinsame Teilchengrößenverteilung für alle in der Probe enthaltenen Teilchenarten (A_{K}) erhalten. Bei Kenntnis des Zusammenhangs zwischen Fluoreszenzintensitätssignal und absoluter Teilchengröße für eine bestimmte in der untersuchten Probe enthaltene Teilchenart, kann man daraus auch eine absolute Teilchengrößenverteilung erhalten. Diese Kenntnis kann in obigem Beispiel der Papierherstellung zur Auswahl und Dosierung eines Zusatzes zur feinverteilten Bindung von Störstoffen an den Papierstoff verwendet werden.

In einer bevorzugten Ausführungsform des erfindungsgemäßen Verfahrens werden Wertepaare (S(Tᵢ),F(Tᵢ)) im Streulichtbereich (SLB) von der Auswertung ausgeschlossen, die über ein für jede Teilchenart (A_{K}) vorgegebenes Maß an Abweichung vom jeweiligen Mittelwert (M(FLB(A_{K}))) abweichen. Diese Eliminierung von vermutlichen oder tatsächlichen Fehlmessungen kann grundsätzlich auf jeder Stufe des Verfahrens stattfinden, bevorzugt aber bei der Zuordnung der Teilchen (Tᵢ) zu einer bestimmten Teilchenart. Stellt man dabei fest, daß das Wertepaar (S(Tⱼ),F(Tⱼ)) eines bestimmten Teilchens (Tⱼ) deutlich außerhalb jedes unterscheidbaren Meßwertgebiets liegt, wird es für die weitere Auswertung zweckmäßigerweise gestrichen. Damit können sich zufällige Meßfehler der Streulicht- und Fluoreszenzlichtmessung nur begrenzt in die Normierungsfaktoren (N(A_{K})) fortpflanzen. Welche Abweichung man bei einem bestimmten Meßwertgebiet, d.h. bei einer bestimmten Teilchenart (A_{K}) als hinnehmbar akzeptiert, hängt von den Umständen des Einzelfalles ab, insbesondere davon, wie genau man die zufälligen Fehler abschätzen kann und wie genau man dementsprechend entscheiden kann, ob ein Meßwert fehlerhaft ist oder nicht.

Vorzugsweise wendet man das erfindungsgemäße Verfahren auf hydrophobe Störstoffteilchen (T(ᵢ)) an. Diese Störstoffpartikel erhält man zum Beispiel, indem man eine Papierstoff- oder Siebwasserprobe aus einer Papiermaschine entnimmt und die freien Störstoffpartikel daraus durch Filtration abtrennt. Die so erhaltenen hydrophoben Störstoffpartikel werden dann mit einem, vorzugsweise lipophilen Fluoreszenzfarbstoff angefärbt, in einem Medium wie Wasser vereinzelt und, wie beschrieben, optisch untersucht.

Im Rahmen des erfindungsgemäßen Verfahrens können die Teilchen (T(ᵢ)) mit mehreren verschiedenen Fluoreszenzfarbstoffen angefärbt sein, wobei die verschiedenen Farbstoffe in verschiedenen Wellenlängenbereichen Fluoreszenzlicht aussenden, das von einem Detektor pro Fluoreszenzbande aufgenommen wird. Diese Farbstoffe können dabei entweder mit der gleichen oder nur mit verschiedenen Anregungsfrequenzen anregbar sein. In letzterem Fall werden dann Lichtquellen mit entsprechend unterschiedlichen Frequenzen eingesetzt, wobei sich die Foki der Lichtquellen entweder überlappen oder nahe beieinander liegen müssen, damit die verschiedenen aufgezeichneten Fluoreszenzsignale auch von dem gleichen Einzelteilchen stammen. Damit können mit Hilfe verschiedener Fluoreszenzfrequenzen die Teilchenarten (A_{K}) noch sicherer voneinander unterschieden werden.

Neben dem beschriebenen Verfahren wird im Rahmen der Erfindung auch eine Vorrichtung zur Bestimmung der Größenverteilung von mindestens zwei Arten (A_{K}) von fluoreszierenden vereinzelten Teilchen (Tᵢ) in einer Probe bereitgestellt. Diese Vorrichtung besitzt mindestens eine Lichtquelle, zum Beispiel einen Laser, die einen fokussierten Lichtstrahl entlang einer Einstrahlungsachse in die Probe sendet, wobei der Fokus des Lichtstrahls vorzugsweise in der Probe liegt, mindestens eine Einrichtung zur Aufnahme mindestens eines Streulichtintensitätswertes (S(Tᵢ)) für jedes Teilchen (Tᵢ), mindestens eine Einrichtung zur Aufnahme mindestens eines Fluoreszenzlichtintensitätswertes (F(Tᵢ)) für jedes Teilchen (Tᵢ), und eine Auswerteeinheit, der die Streulichtintensitätswerte (S(Tᵢ)) und die Fluoreszenzlichtintensitätswerte (F(Tᵢ)) für jedes Teilchen (Tᵢ) zugeleitet werden, und die derart gestaltet ist, daß sie mindestens folgende Auswerteschritte ausführen kann:
(a) Zuordnung der Teilchen (Tᵢ) zu einer Teilchenart (A_{K}) mit Hilfe der Lage ihrer Wertepaare (S(Tᵢ),F(Tᵢ)) in einem Bereich (B_{K}) in einem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, wobei jeder Bereich (B_{K}) mindestens ein lokales Maximum der Häufigkeit der Wertepaare (S(Tᵢ), F(Tᵢ)) in dem Raum (R) für die Teilchenart (A_{K}) aufweist;
(b) Ermittlung der relativen Häufigkeit der Fluoreszenzlichtintensitätswerte (F(Tᵢ)) für jede Teilchenart (A_{K});
(c) Berechnung der relativen Teilchengrößenverteilung für jede Teilchenart (A_{K}) aus der relativen Häufigkeit der Fluoreszenzlichtintensitätswerte (F(Tᵢ)) für die entsprechende Teilchenart (A_{K});
(d) Normierung der relativen Teilchengrößenverteilungen für die einzelnen Teilchenarten (A_{K}) mit Hilfe der relativen Lage der Bereiche (B_{K}) in dem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, aufeinander; und
(e) Bildung einer gemeinsamen relativen Teilchengrößenverteilung für alle Teilchenarten (A_{K}).

Bevorzugt ist eine Vorrichtung mit einer Auswerteeinheit (23,24,25), die zur Normierung der relativen Teilchengrößenverteilungen für die einzelnen Teilchenarten (A_{K}) aufeinander in Schritt (d) noch mindestens folgende Schritte ausführen kann:
(a) Wahl eines Streulichtbereiches (SLB(A_{K})) von Streulichtintensitätswerten (S(Tᵢ)) mit einer vorbestimmten Größe für jede Teilchenart (A_{K}), in dem die Häufigkeit der Wertepaare (S(Tᵢ),F(Tᵢ)) der Teilchenart (A_{K}) mindestens ein lokales Maximum aufweist;
(b) Bestimmung eines Streulichtbereiches (SLB) von Streulichtintensitätswerten (S(Tᵢ)) von vorbestimmter Größe mit einer oberen und einer unteren Bereichsgrenze, dessen Mittelwert aus der oberen und der unteren Bereichsgrenze gleich dem Mittelwert der Mittelwerte aus den Streulichtintensitätswerten (S(Tᵢ)) in den jeweiligen Streulichtbereichen (SLB(A_{K})) ist. Wie bereits beim Verfahren oben beschrieben, können die Schritte (a) und (b) auch durch einen einzigen Schritt ersetzt werden, in dem ein Streulichtbereich (SLB) ohne vorherige Bestimmung der teilchenartspezifischen Streulichtbereiche (SLB(A_{K})) gewählt wird. Dabei gelten für die Auswahl der Größe und Lage des Streulichtbereichs (SLB) die obigen Anmerkungen;
(c) Bestimmung eines Fluoreszenzlichtbereiches (FLB(A_{K})) von Fluoreszenzlichtintensitätswerten (F(Tᵢ)) mit vorbestimmter Größe für jede Teilchenart (A_{K}), dessen Wertepaare (S(Tᵢ),F(Tᵢ)) auch in den Streulichtbereich (SLB) fallen;
(d) Bestimmung des Mittelwerts (M(FLB(A_{K}))) der Fluoreszenzlichtintensitätswerte (F(Tᵢ)) in dem Fluoreszenzlichtbereich (FLB(A_{K})) für jede Teilchenart (A_{K});
(e) Bildung eines Normierungsfaktors (N(A_{K})) für jede Teilchenart (A_{K}), bezogen auf eine beliebige Teilchenart (A₁), wobei gilt: (N(A_{K})) = (M(FLB(A_{K})))/(M(FLB(A₁))); und
(f) In-Beziehung-Setzen der relativen Teilchengrößenverteilungen der Teilchenarten (A_{K}) zueinander mit Hilfe der Normierungsfaktoren (N(A_{K})).
   Die oben zu den entsprechenden Schritten des erfindungsgemäßen Verfahrens gegebenen Erläuterungen gelten hier sinngemäß.

Vorzugsweise ist die Einrichtung zur Aufnahme mindestens eines Streulichtintensitätswertes (S(Tᵢ)) für jedes Teilchen (Tᵢ) derart ausgestaltet und in der Vorrichtung angeordnet, daß die Streulichtintensitätswerte (S(Tᵢ)) in einem Hohlkegel aufgenommen werden, dessen innere Mantelfläche mit der Einstrahlungsachse der Lichtquelle (10) einen Winkel von mindestens 5°, und dessen äußere Mantelfläche mit dieser Achse einen Winkel von höchstens 50° einschließt.

Bevorzugt ist auch eine Vorrichtung mit einer Auswerteeinheit, die solche Wertepaare (S(Tᵢ), F(Tᵢ)) im Streulichtbereich (SLB) von der Auswertung ausschließt, die über ein für jede Teilchenart (A_{K}) vorgegebenes Maß an Abweichung vom jeweiligen Mittelwen (M(FLB(A_{K}))) abweichen. Welche Abweichung dabei als tolerabel, d.h. als wahrscheinlich nicht auf einem Meßfehler beruhend angesehen wird, hängt von den Umständen des Einzelfalles ab. Diesbezüglich wird wieder auf die entsprechenden Erläuterungen zum Verfahren verwiesen.

Das obige erfindungsgemäße Verfahren und die beschriebene Vorrichtung sind vorzugsweise zur Bestimmung der Teilchengrößenverteilung von Teilchen bei der Papierherstellung, insbesondere von Störstoffteilchen, vor allem hydrophoben Störstoffteilchen, im Papierstoff oder im Siebwasser von Papiermaschinen geeignet. Insbesondere können sie dazu verwendet werden, die Zudosierung von Zusätzen zu Papierstoff oder Siebwasser von Papiermaschinen zu steuern oder zu regeln, insbesondere von solchen Zusätzen, die dazu dienen hydrophobe Störstoffteilchen unschädlich zu machen, um Ablagerungen in Papiermaschinen zu vermeiden. Diese Steuerung oder Regelung erfolgt aufgrund eines Steuersignals, das als Resultat der gemeinsamen relativen Teilchengrößenverteilung für die verschiedenen Störstoffteilchen ausgegeben wird. Bei der Detektion von größeren Teilchen kann etwa eine Pumpe bzw. ein Ventil mehr oder andere Zusätze beigeben. Damit kann die Produktqualität bei schwankender Zusammensetzung von Störstoffen gewahrt bleiben.

Die vorliegende Erfindung wird im folgenden anhand der Figuren 1 bis 5 beschrieben. Dabei zeigen:
- Figur 1:: Schematische Darstellung einer Vorrichtung zur Vereinzelung von Teilchen in einer Probe.
- Figur 2:: Blockdiagramm einer Vorrichtung zur Bestimmung der Teilchengrößenverteilung in einer Probe gemäß der Erfindung.
- Figur 3:: Diagramm der Meßergebnisse für die Streulichtintensität und die Fluoreszenzlichtintensität bei einer Beispielsmessung mit zwei Teilchenarten in der Probe.
- Figur 4a:: Diagramm der Verteilung der Fluoreszenzintensitätswerte für die Beispielsmessung nach Figur 3.
- Figur 4b:: Diagramm der relativen Teilchengrößenverteilung für die erste Teilchenart der Beispielsmessung von Figur 3.
- Figur 4c:: Diagramm der relativen Teilchengrößenverteilung für die zweite Teilchenart der Beispielsmessung von Figur 3.
- Figur 5:: Diagramm der gemeinsamen relativen Teilchengrößenverteilung für beide Teilchenarten der Beispielsmessung von Figur 3.

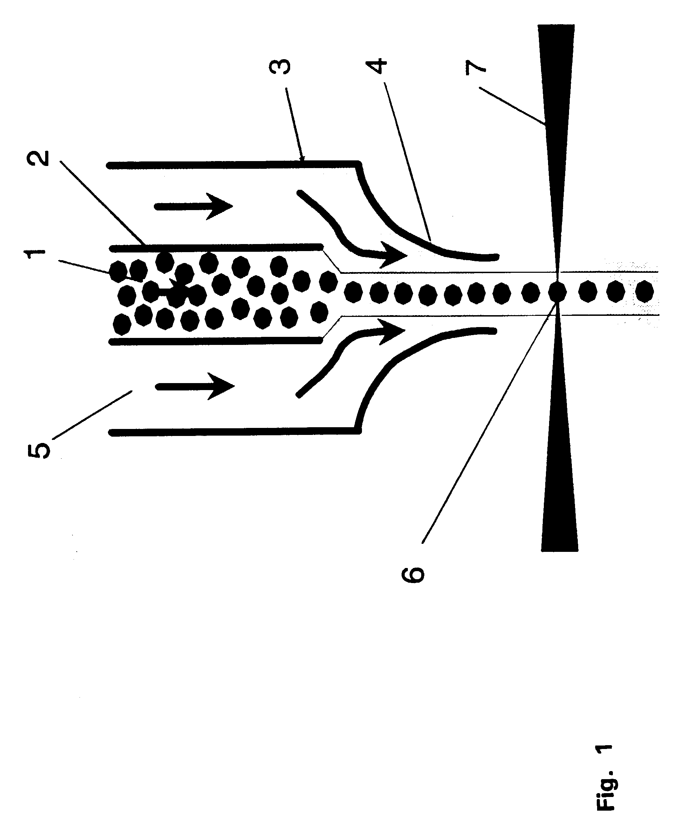

Figur 1 zeigt eine Vorrichtung zur Vereinzelung von Teilchen in einer Probe. Eine Vereinzelung der optisch zu untersuchenden Teilchen ist im Rahmen der vorliegenden Erfindung notwendig, um sicher sein zu können, daß jeder einzelne Meßwert, also jedes Wertepaar (S(Tᵢ),F(Tᵢ)) zu einem bestimmten Teilchen Tᵢ gehört. So können im Rahmen des Verfahrens der vorliegenden Erfindung die Einflüsse unterschiedlicher Anfärbbarkeit und Quantenausbeute abgeschätzt und beseitigt werden. In der Vorrichtung nach Figur 1 wird ein Probenstrom 1 mit den zu untersuchenden Teilchen durch eine Kapillare 2 in eine Hüllstromküvette 3 geleitet, an deren Ende sich eine Düse 4 befindet. Über eine die Kapillare 2 umgebende hohlzylindrische Leitung 5 wird ein Hüllstrom, zum Beispiel einfach Wasser, in die Küvette 3 eingebracht. Dabei hat der Hüllstrom aus der Leitung 5 eine deutlich höhere Geschwindigkeit als der Probenstrom 1 in der Kapillare 2. Am Ende der Kapillare 2 vermischen sich der Probenstrom 1 und der Hüllstrom aus der Leitung 5, wobei aufgrund der höheren Geschwindigkeit des Hüllstroms die Partikel in dem Probenstrom 1 über eine weitere Strecke verteilt, der Probenstrom also bezüglich der zu untersuchenden Teilchen verdünnt wird. Dieses Prinzip wird als hydrodynamische Fokussierung bezeichnet. Der aus der Düse 4 austretende verdünnte Probenstrahl enthält die Probenteilchen somit praktisch völlig vereinzelt. Wenn man dann einen fokussierten Lichtstrahl 7, zum Beispiel einen Laserstrahl auf einen beliebigen Meßort 6 in diesem Strahl richtet, betrachtet man fast immer ein einzelnes Teilchen im Probenstrom. Im Rahmen der vorliegenden Erfindung ist dieses Prinzip der hydrodynamischen Fokussierung zur Vereinzelung der Teilchen in der Probe besonders geeignet.

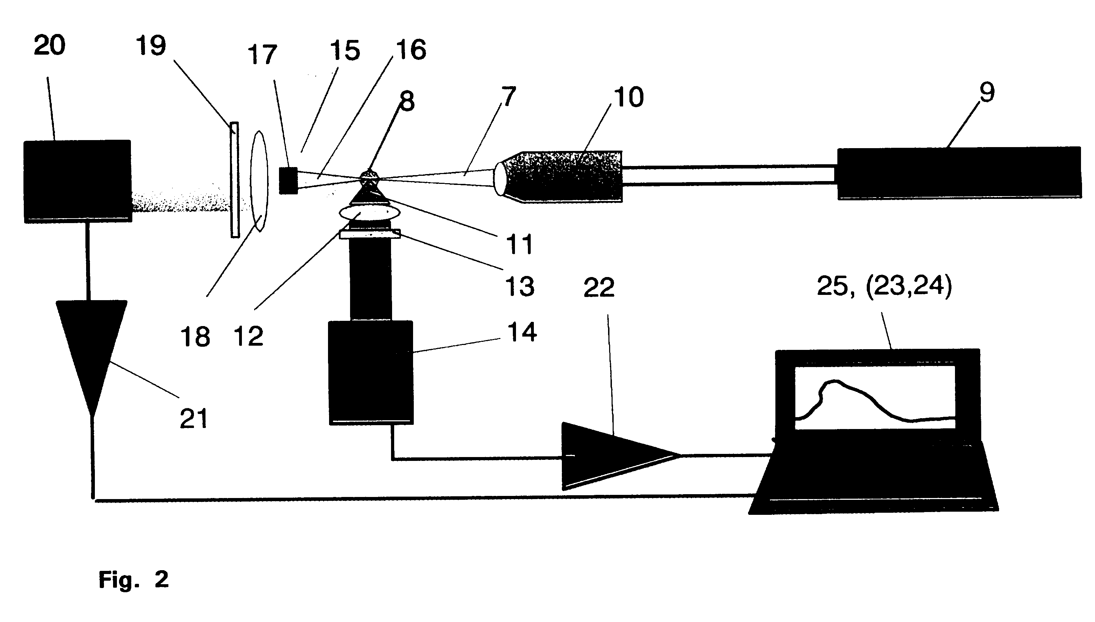

Figur 2 zeigt eine Prinzipskizze eines Meßaufbaus nach der vorliegenden Erfindung. Ein Laser 9 liefert Anregungslicht an ein Objektiv 10, das das Laserlicht auf eine Probe 8 fokussiert. Der Fokus liegt dabei vorzugsweise in der Probe 8, er kann aber auch außerhalb derselben liegen. Wesentlich ist dabei nur, daß die Intensität des Anregungslichts in der Probe genügend hoch ist und daß der Lichtkegel des Anregungslichts nicht so breit ist, daß mehrere Probenteilchen zugleich angeregt werden. Ein Photomultiplier 20 fängt über eine Linse 18 das in der Probe 8 vorwärts gestreute Anregungslicht auf und leitet die Streulichtintensitätswerte oder dazu proportionale elektrische Signale über einen Verstärker 21 an einen Computer 25 weiter. Dem Photomultiplier 20 ist hier ein Strahlstopper 17 und ein Kantenfilter 19 vorgeschaltet, ersterer vor, letzterer nach der Linse 18. Der Strahlstopper 17 hat die Funktion, den intensiven Anteil von ungestreut durchgehendem Anregungslicht in dem von der Probe 8 kommenden kegelförmigen Streulichtstrahl 16 herauszufiltern. Vorzugsweise wird dabei ungefähr ein Kernkegel mit einem Öffnungswinkel von 5° herausgefiltert. Die Messung des Streulichts geschieht im übrigen bevorzugt in einem Hohlkegel, dessen innere Mantelfläche mit der Kegelachse einen Winkel von mindestens 5°, und dessen äußere Mantelfläche mit der Kegelachse einen Winkel von höchstens 50° einschließt. Entsprechend weist die erfindungsgemäße Vorrichtung nach Figur 2 einen Photomultiplier 14 zur Registrierung des Fluoreszenzlichts 11 aus der Probe 8 auf. Wie in Figur 2 gezeigt, wird das Fluoreszenzlicht aus der Probe vorzugsweise in einer 90°-Richtung zum Einfallslichtstrahl aufgenommen. Im Strahlengang zur Aufnahme des Fluoreszenzlichts befindet sich auch noch eine Linse 12 und ein Kantenfilter 13. Der Photomultiplier 14 leitet Fluoreszenzlichtintensitätssignale über einen Verstärker 22 an den Computer 25. Dieser enthält je einen Vielkanalanalysator 23 und 24 für das Streulicht und für das Fluoreszenzlicht, der die Intensitätswerte sortiert.

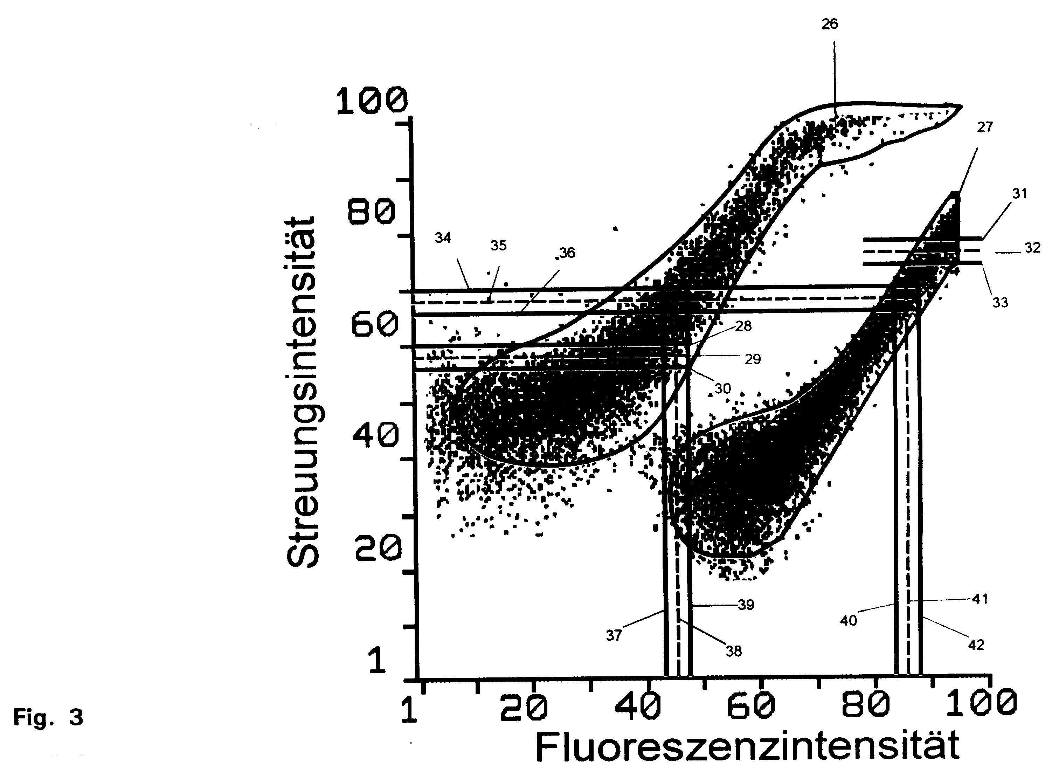

Für die den folgenden Figuren 3 bis 5 zugrundeliegende Beispielsmessung mit der erfindungsgemäßen Vorrichtung wurde eine Siebwasserprobe aus einer Papiermaschine entnommen und filtriert, so daß die hydrophoben Störstoffteilchen weitgehend abgetrennt wurden. Diese Filterung wurde mit einem sog. Dynamic Drainage Jar ausgeführt, hier einem Plexiglasbehälter mit eingebautem Papiersieb mit einer Maschenweite von 40 *µ*m. Dabei wurde der Papierbrei zuerst auf etwa 0,4% Feststoffgehalt verdünnt und dann unter Rühren über das Sieb abfiltriert. Das Filtrat, das Fasern und Störstoffteilchen mit einer Größe unter 40 *µ*m enthielt, wurde dann nochmals im Verhältnis 1:10 verdünnt und mit einer Farbstofflösung im Verhältnis 1:25 angefärbt. Diese Farbstofflösung enthielt Wasser und 40 mg/l Ethanol und war mit dem im Handel erhältlichen Fluoreszenzfarbstoff "Fluorol 555" von BASF, d.i. N-(n-butyl)-4-(n-butylamino)-Naphthalsäureimid übersättigt. Die Anfärbezeit betrug etwa 2 Minuten. Damit wurde erreicht, daß nahezu alle hydrophoben Störstoffteilchen, nicht aber die im Filtrat verbliebenen kleinen Papierfasern angefärbt wurden.

Anschließend wurden die angefärbten Störstoffteilchen aus der Farbstofflösung abgefiltert und in einer Vorrichtung, wie sie in Figur 1 beschrieben wurde, hydrodynamisch in einem Wasserstrahl vereinzelt. Dabei hatte die verwendete Küvette einen Durchmesser von 100 *µ*m, die Düse eine Durchmesser von 200 *µ*m. Die Hüllstromgeschwindigkeit war etwa 100 mal so groß wie die Probengeschwindigkeit.

Dann wurden die vereinzelten Störstoffteilchen mit einer Meßapparatur, wie sie in Figur 2 beschrieben wurde, untersucht. Die Ergebnisse sind in den folgenden Figuren 3 bis 5 dargestellt.

Figur 3 zeigt den Auftrag der gemessenen Streulichtintensitätswerte über den zugehörigen Fluoreszenzlichtintensitätswerten für eine Beispielsmessung mit einer erfindungsgemäßen Vorrichtung an den obigen Störstoffteilchen. Die Koordinatenachsen des Diagramms von Figur 3 geben relative Werte an. Aus der Figur 3 kann man ersehen, daß sich die Wertepaare (S(Tᵢ), F(Tᵢ)) für die Streulichtintensität und für die Fluoreszenzlichtintensität der Teilchen Tᵢ in deutlich unterscheidbare Meßwertbereiche B_{K} ordnen, die hier als keulenförmige Bereiche 26,27 erscheinen. Jeder dieser Bereiche B_{K} entspricht einem Störstoff, also einer Teilchenart A_{K}. Um fehlerhafte Meßwerte von der weiteren Auswertung auszuschließen, begrenzt man die Meßwertbereiche, die hier als durchgehende Linien um die "Keulen" 26 und 27 dargestellt sind. Das Kriterium zur Ziehung der Linien um die "Keulen" 26,27, also zum Ausschluß von Meßwerten kann dabei ein strikt mathematisches Kalkül oder auch das Ermessen des erfahrenen Experimentators sein. Im nächsten Schritt ermittelt man die Bereiche größter Meßwertdichte bzw. größter Meßwerthäufigkeit in den "Keulen" 26,27 und wählt für die beiden Teilchenarten A₁ und A₂ einen zugehörigen Streulichtbereich SLB(A₁), hier durch die Linien 28 und 30 begrenzt, und SLB(A₂), hier durch die Linien 31 und 33 begrenzt, wobei diese Bereiche von vorgegebener Größe sind. Dann ermittelt man die beiden Mittelwerte 29 und 32 der Streulichtintensitätswerte S(Tᵢ) in den beiden Streulichtbereichen SLB(A₁) und SLB(A₂). Der Mittelwert aus den beiden Mittelwerten 29 und 32 soll dann als Mittelwert 35 für den gemeinsamen Streulichtbereich SLB, hier durch die Linien 34 und 36 begrenzt, dienen, der sich symmetrisch um den Mittelwert 35 parallel zur Koordinatenachse der Fluoreszenzintensitätswerte erstreckt. Dieser Streulichtbereich SLB besitzt wiederum, wie die anderen definierten Streulichtbereiche SLB(A₁) und SLB(A₂) eine vorgegebene Größe, d.h. eine vorgegebene Differenz des Streulichtintensitätswerts an der oberen Bereichsgrenze 34 und an der unteren Bereichsgrenze 36. In einem nächsten Schritt werden Fluoreszenzlichtbereiche FLB(A₁), hier begrenzt durch die Linien 37 und 39, und FLB(A₂), hier begrenzt durch die Linien 40 und 42, bestimmt, wobei die Wertepaare (S(Tᵢ),F(Tᵢ)) der Fluoreszenzlichtbereiche FLB(A₁) und FLB(A₂) auch in den Streulichtbereich SLB, hier begrenzt durch die Linien 34 und 36, fallen. Dann werden die Mittelwerte M(FLB(A₁), hier als Linie 38 dargestellt, und M(FLB(A₂), hier als Linie 41 dargestellt, der Fluoreszenzlichtintensitätswerte F(Tᵢ) in beiden Fluoreszenzlichtbereichen FLB(A₁) und FLB(A₂) ermittelt. Aus diesen Mittelwerten wird schließlich für die Teilchenart A₂ ein Normierungsfaktor N(A₂) errechnet, wobei N(A₂) der Quotient aus M(FLB(A₂) und M(FLB(A₁) ist, also gilt: N(A₂) = M(FLB(A₂)/M(FLB(A₁).

Figur 3 ist gewissermaßen eine zweidimensionale Projektion des Raumes R, der aus den Streulichtintensitätswerten S(Tᵢ), den Fluoreszenzlichtintensitätswerten F(Tᵢ) und der Häufigkeit der Wertepaare (S(Tᵢ),F(Tᵢ)) aufgespannt wird. Jeder der Bereiche B_{K} enthält mindestens ein lokales Maximum der Wertepaarhäufigkeit, und damit auch der Wertepaardichte, für die zugehörige Teilchenart A_{K}. Statt der beschriebenen Auswerteart können die "Keulen" auch anders ausgewertet werden, etwa durch Ermittlung ihres parallelen Abstandes im Diagramm nach Figur 3 oder durch jede beliebige andere Rechenregel, soweit sie den physikalischen Gegebenheiten der Messung hinreichend Rechnung trägt.

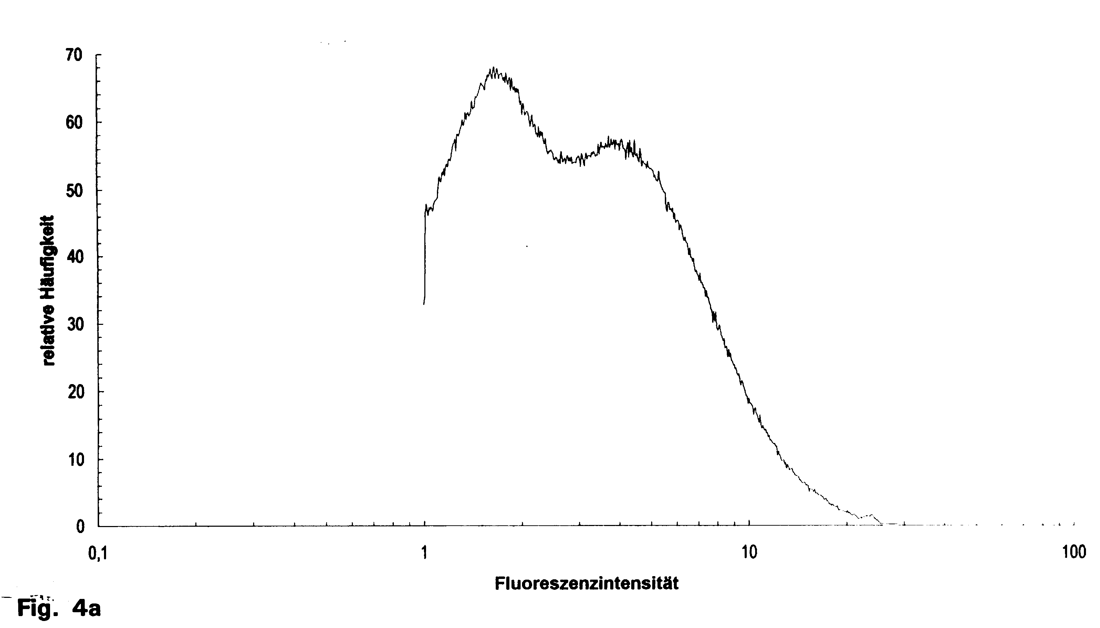

Figur 4a zeigt die Häufigkeit der Fluoreszenzintensitätswerte für die in Figur 3 diskutierte Beispielsmessung für beide Partikelsorten A₁ und A₂. Daraus kann man wegen der unterschiedlichen Anfärbbarkeit und Quantenausbeute der verschiedenen Teilchenarten A₁ und A₂ keine zuverlässige Aussage über die gemeinsame Teilchengrößenverteilung der verschiedenen Probenpartikel treffen.

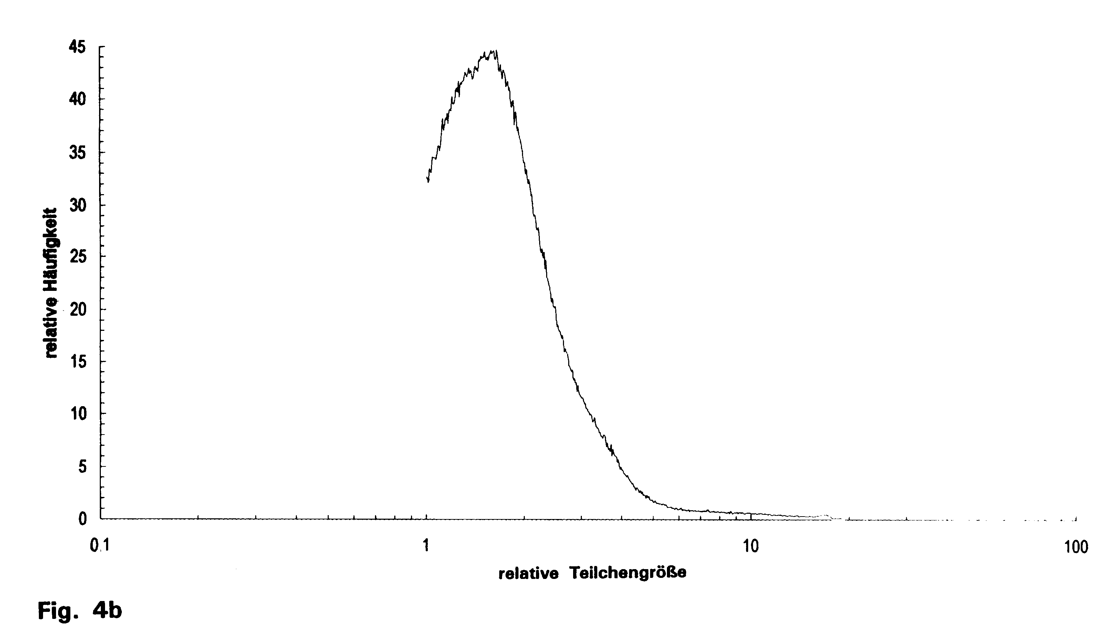

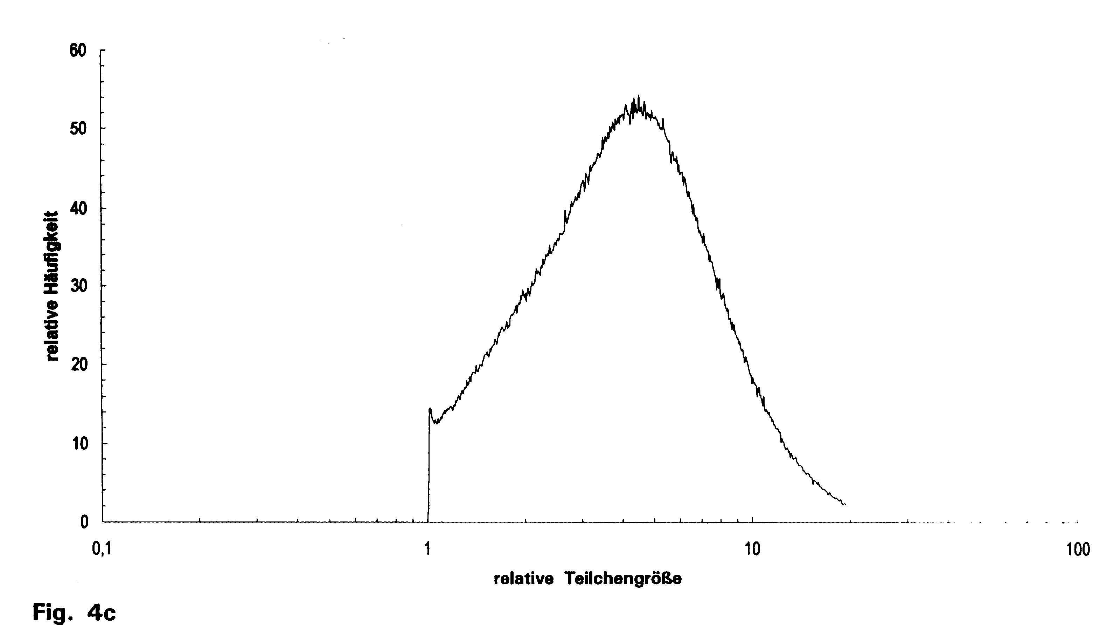

Figuren 4b und 4c zeigen die relativen Teilchengrößenverteilungen (in beliebigen Einheiten), die man für die Teilchenarten A₁ und A₂ nach deren Trennung über die in Figur 3 dargestellte Zuordnung zu einem Meßwertbereich, d.h. einer "Keule" erhält.

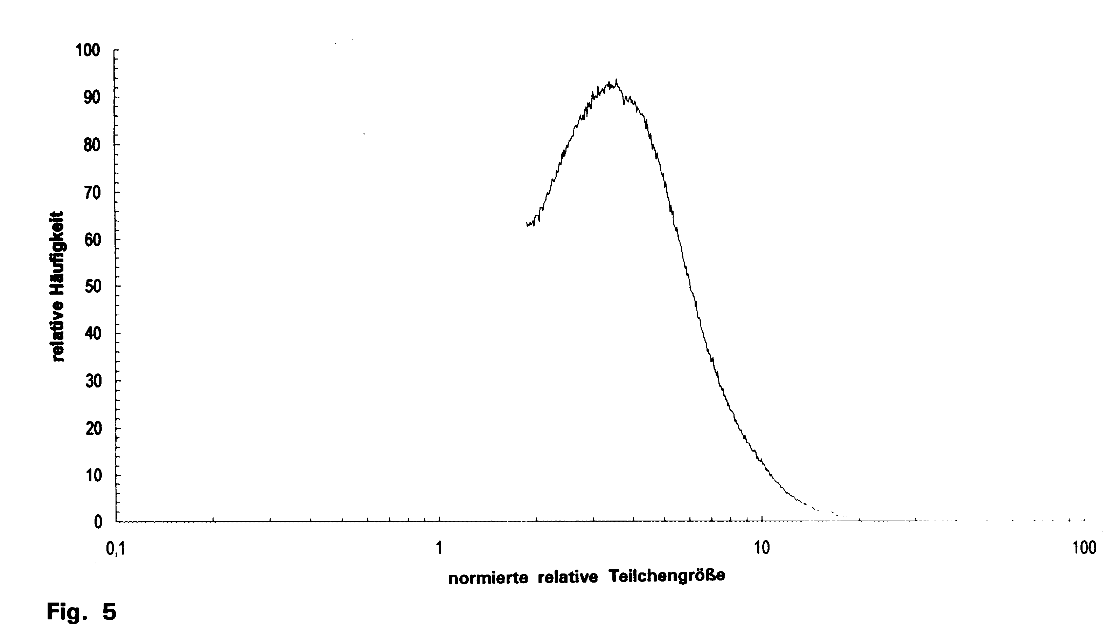

Figur 5 schließlich zeigt das Ergebnis des erfindungsgemäßen Verfahrens, die teilchenartunabhängige Teilchengrößenverteilung, die man durch eine Normierung der relativen Teilchengrößenverteilung der Teilchenart A₂ auf die relative Teilchengrößenverteilung der Teilchenart A₁ mit dem Normierungsfaktor N(A₂) erhält. In Figur 5 ist eine gemeinsame relative, also eine normierte relative Teilchengrößenverteilung dargestellt. Man kann aber auch eine gemeinsame absolute Teilchengrößenverteilung errechnen, wenn mindestens eine der beiden relativen Teilchengrößenverteilungen für die verschiedenen Partikelsorten A₁, A₂ auf absolute Teilchengrößen geeicht ist.

Die vorliegende Erfindung bietet somit ein Verfahren und eine Vorrichtung zur Bestimmung der relativen und absoluten Teilchengrößenverteilung von verschiedenen Partikeln in einer Probe, das einfach und schnell und damit in besonderem Maße für den Online-Betrieb geeignet ist.

## Patentansprüche

1. Verfahren zur Bestimmung der Größenverteilung von mindestens zwei Teilchenarten (A_{K}) von fluoreszierenden Teilchen (Tᵢ) in einer Probe, wobei
die Teilchen (Tᵢ) in der Probe vereinzelt werden und Licht in die Probe entlang einer vorgegebenen Einstrahlungsrichtung eingestrahlt wird,
mindestens ein Streulichtintensitätswert (S(Tᵢ)) und mindestens ein Fluoreszenzlichtintensitätswert (F(Tᵢ)) von jedem Teilchen (Tᵢ) gemessen wird,
die Teilchen (Tᵢ) aufgrund der Lage ihrer Wertepaare (S(Tᵢ),F(Tᵢ)) in einem Bereich (B_{K}) in einem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, jeweils einer Teilchenart (A_{K}) zugeordnet werden, wobei jeder Bereich (B_{K}) mindestens ein lokales Maximum der Häufigkeit der Wertepaare (S(Tᵢ),F(Tᵢ)) in dem Raum (R) für die Teilchenart (A_{K}) aufweist,
die relative Häufigkeit der Fluoreszenzlichtintensitätswerte (F(Tᵢ)) für jede Teilchenart (A_{K}) ermittelt wird,
die relative Teilchengrößenverteilung für jede Teilchenart (A_{K}) aus der relativen Häufigkeit der Fluoreszenzlichtintensitätswerte (F(Tᵢ)) für die entsprechende Teilchenart (A_{K}) berechnet wird,
die relativen Teilchengrößenverteilungen für die einzelnen Teilchenarten (A_{K}) mit Hilfe der Lage der Bereiche (B_{K}) in dem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, aufeinander normiert werden, und
damit eine gemeinsame relative Teilchengrößenverteilung für die Teilchen (Tᵢ) aller Teilchenarten (A_{K}) gebildet wird.

2. Verfahren nach Anspruch 1, dadurch gekennzeichnet, daß für die Normierung der relativen Teilchengrößenverteilungen für die einzelnen Teilchenarten (A_{K})
ein Streulichtbereich (SLB) von Streulichtintensitätswerten (S(Tᵢ)) von vorbestimmter Größe mit einer oberen und einer unteren Bereichsgrenze gewählt wird, in dem die Häufigkeit der Wertepaare (S(Tᵢ),F(Tᵢ)) für alle Teilchenarten (A_{K}) mindestens ein lokales Maximum aufweist,
ein Fluoreszenzlichtbereich (FLB(A_{K})) von Fluoreszenzlichtintensitätswerten (F(Tᵢ)) für jede Teilchenart (A_{K}) von vorbestimmter Größe bestimmt wird, dessen Wertepaare (S(Tᵢ),F(Tᵢ)) auch in den Streulichtbereich (SLB) fallen,
ein Mittelwert (M(FLB(A_{K}))) der Fluoreszenzlichtintensitätswerte (F(Tᵢ)) in dem Fluoreszenzlichtbereich (FLB(A_{K})) für jede Teilchenart (A_{K}) bestimmt wird,
für jede Teilchenart (A_{K}) ein Normierungsfaktor (N(A_{K})), bezogen auf eine Teilchenart (A₁) gebildet wird, wobei (N(A_{K})) = (M(FLB(A_{K})))/(M(FLB(A₁))) ist, und
die relativen Teilchengrößenverteilungen der Teilchenarten (A_{K}) mit Hilfe der Normierungsfaktoren (N(A_{K})) zueinander in Beziehung gesetzt werden.

3. Verfahren nach Anspruch 2, dadurch gekennzeichnet, daß der Streulichtbereich (SLB) dadurch bestimmt wird, daß jeweils ein Streulichtbereich (SLB(A_{K})) von Streulichtintensitätswerten (S(Tᵢ)) für jede Teilchenart (A_{K}) von vorbestimmter Größe gewählt wird, in dem die Häufigkeit der Wertepaare (S(Tᵢ),F(Tᵢ)) für die Teilchenart (A_{K}) mindestens ein lokales Maximum aufweist, und der Mittelwert aus der oberen und der unteren Bereichsgrenze des Streulichtbereichs (SLB) gleich dem Mittelwert der Mittelwerte aus den Streulichtintensitätswerten (S(Tᵢ)) in den Streulichtbereichen (SLB(A_{K})) gesetzt wird.

4. Verfahren nach einem der vorhergehenden Ansprüche, dadurch gekennzeichnet, daß diejenigen Wertepaare (S(Tᵢ), F(Tᵢ)) im Streulichtbereich (SLB) von der Auswertung ausgeschlossen sind, die über ein für jede Teilchenart (A_{K}) vorgegebenes Maß an Abweichung vom jeweiligen Mittelwert (M(FLB(A_{K}))) abweichen.

5. Verfahren nach einem der vorhergehenden Ansprüche, dadurch gekennzeichnet, daß die Vereinzelung der Teilchen (Tᵢ) durch hydrodynamische Fokussierung erfolgt.

6. Verfahren nach einem der vorhergehenden Ansprüche, dadurch gekennzeichnet, daß die Teilchen (Tᵢ) mit mindestens einem, vorzugsweise lipophilen Fluoreszenzfarbstoff markiert sind.

7. Verfahren nach einem der vorhergehenden Ansprüche, dadurch gekennzeichnet, daß die aufgenommenen Streulichtintensitätswerte (S(Tᵢ)) in einem Vorwärtsstreuhohlkegel liegen, dessen innere Mantelfläche mit der Einstrahlungsrichtung des Lichts in die Probe einen Winkel von mindestens 5°, und dessen äußere Mantelfläche mit dieser Richtung einen Winkel von höchstens 50° einschließt.

8. Vorrichtung zur Bestimmung der Größenverteilung von mindestens zwei Arten (A_{K}) von fluoreszierenden vereinzelten Teilchen (Tᵢ) in einer Probe (8), gekennzeichnet durch
mindestens eine Lichtquelle (9,10), die einen fokussierten Lichtstrahl (7) entlang einer Einstrahlungsachse in die Probe (8) sendet,
mindestens eine Einrichtung (17,18,19,20,21) zur Aufnahme mindestens eines Streulichtintensitätswertes (S(Tᵢ)) für jedes Teilchen (Tᵢ),
mindestens eine Einrichtung (12,13,14,22) zur Aufnahme mindestens eines Fluoreszenzlichtintensitätswertes (F(Tᵢ)) für jedes Teilchen (Tᵢ),
eine Auswerteeinheit (23,24,25), der die Streulichtintensitätswerte (S(Tᵢ)) und die Fluoreszenzlichtintensitätswerte (F(Tᵢ)) für jedes Teilchen (Tᵢ) zugeleitet werden, und die derart ausgestaltet ist, daß sie mindestens folgende Auswerteschritte ausführen kann:
(a) Zuordnung der Teilchen (Tᵢ) zu einer Teilchenart (A_{K}) mit Hilfe der Lage ihrer Wertepaare (S(Tᵢ),F(Tᵢ)) in einem Bereich (B_{K}) in einem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, wobei jeder Bereich (B_{K}) mindestens ein lokales Maximum der Häufigkeit der Wertepaare (S(Tᵢ), F(Tᵢ)) in dem Raum (R) für die Teilchenart (A_{K}) aufweist;
(b) Ermittlung der relativen Häufigkeit der Fluoreszenzlichtintensitätswerte (F(Tᵢ)) für jede Teilchenart (A_{K});
(c) Berechnung der relativen Teilchengrößenverteilung für jede Teilchenart (A_{K}) aus der relativen Häufigkeit der Fluoreszenzlichtintensitätswerte (F(Tᵢ)) für die jeweilige Teilchenart (A_{K});
(d) Normierung der relativen Teilchengrößenverteilungen für die einzelnen Teilchenarten (A_{K}) mit Hilfe der Lage der Bereiche (B_{K}) in dem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, aufeinander; und
(e) Bildung einer gemeinsamen relativen Teilchengrößenverteilung für alle Teilchenarten (A_{K}).

9. Vorrichtung nach Anspruch 8, gekennzeichnet durch eine Auswerteeinheit (23,24,25), die zur Normierung der relativen Teilchengrößenverteilungen für die einzelnen Teilchenarten (A_{K}) aufeinander in Schritt (d) noch mindestens folgende Schritte ausführen kann:
(a) Wahl eines Streulichtbereiches (SLB) von Streulichtintensitätswerten (S(Tᵢ)) von vorbestimmter Größe mit einer oberen und einer unteren Bereichsgrenze, in dem die Häufigkeit der Wertepaare (S(Tᵢ),F(Tᵢ)) für alle Teilchenarten (A_{K}) mindestens ein lokales Maximum aufweist;
(b) Bestimmung eines Fluoreszenzlichtbereiches (FLB(A_{K})) von Fluoreszenzlichtintensitätswerten (F(Tᵢ)) mit vorbestimmter Größe für jede Teilchenart (A_{K}), dessen Wertepaare (S(Tᵢ),F(Tᵢ)) auch in den Streulicht-bereich (SLB) fallen;
(c) Bestimmung des Mittelwerts (M(FLB(A_{K}))) der Fluoreszenzlichtintensitätswerte (F(Tᵢ)) in dem Fluoreszenzlichtbereich (FLB(A_{K})) für jede Teilchenart (A_{K});
(d) Bildung eines Normierungsfaktors (N(A_{K})) für jede Teilchenart (A_{K}), bezogen auf eine beliebige Teilchenart (A₁), wobei gilt: (N(A_{K})) = (M(FLB(A_{K})))/(M(FLB(A₁))); und
(e) In-Beziehung-Setzen der relativen Teilchengrößenverteilungen der Teilchenarten (A_{K}) zueinander mit Hilfe der Normierungsfaktoren (N(A_{K})).

10. Vorrichtung nach Anspruch 9, gekennzeichnet durch eine Auswerteeinheit (23,24,25), die derart ausgestaltet ist, daß sie in Schritt (a) einen Streulichtbereich (SLB) dadurch bestimmen kann, daß sie jeweils einen Streulichtbereich (SLB(A_{K})) von Streulichtintensitätswerten (S(Tᵢ)) mit einer vorbestimmten Größe für jede Teilchenart (A_{K}) wählt, in dem die Häufigkeit der Wertepaare (S(Tᵢ),F(Tᵢ)) für die jeweilige Teilchenart (A_{K}) mindestens ein lokales Maximum aufweist, und den Mittelwert aus der oberen und der unteren Bereichsgrenze des Streulichtbereichs (SLB) gleich dem Mittelwert der Mittelwerte aus den Streulichtintensitätswerten (S(Tᵢ)) in den jeweiligen Streulichtbereichen (SLB(A_{K})) setzt.

11. Vorrichtung nach einem der vorhergehenden Vorrichtungsansprüche, dadurch gekennzeichnet, daß die Lichtquelle (9,10) einen Laser enthält.

12. Vorrichtung nach einem der vorhergehenden Vorrichtungsansprüche, dadurch gekennzeichnet, daß die Einrichtung (17,18,19,20,21) zur Aufnahme mindestens eines Streulichtintensitätswertes (S(Tᵢ)) für jedes Teilchen (Tᵢ) derart ausgestaltet und angeordnet ist, daß die Streulicht-intensitätswerte (S(Tᵢ)) in einem Hohlkegel aufgenommen werden, dessen innere Mantelfläche mit der Einstrahlungsachse der Lichtquelle (9,10) einen Winkel von mindestens 5°, und dessen äußere Mantelfläche mit dieser Achse einen Winkel von höchstens 50° einschließt.

13. Vorrichtung nach einem der vorhergehenden Vorrichtungsansprüche, gekennzeichnet durch eine Auswerteeinheit (23,24,25), die Wertepaare (S(Tᵢ),F(Tᵢ)) im Streulichtbereich (SLB) von der Auswertung ausschließt, die über ein für jede Teilchenart (A_{K}) vorgegebenes Maß an Abweichung vom jeweiligen Mittelwert (M(FLB(A_{K}))) abweichen.

14. Verwendung eines Verfahrens nach einem der Ansprüche 1 bis 7 oder einer Vorrichtung nach einem der Ansprüche 8 bis 13 zur Bestimmung der Teilchengrößenverteilung von Teilchen bei der Papierherstellung.

15. Verwendung nach Anspruch 14, dadurch gekennzeichnet, daß die Teilchengrößenverteilung von Störstoffteilchen, insbesondere hydrophoben Störstoffteilchen, im Papierstoff oder im Siebwasser von Papiermaschinen bestimmt wird.

16. Verwendung nach Anspruch 14 oder 15 zur Steuerung der Zudosierung von Zusätzen zum Papierstoff oder Siebwasser von Papiermaschinen, insbesondere von solchen Zusätzen, die dazu dienen hydrophobe Störstoffteilchen unschädlich zu machen, um Ablagerungen in Papiermaschinen zu vermeiden, wobei ein der gemeinsamen relativen Teilchengrößenverteilung entsprechendes bzw. zugeordnetes Steuersignal erzeugt wird und die Zudosierung aufgrund dieses Steuersignals gesteuert wird.
